(19) Europäisches Patentamt
European Patent Office
Office européen des brevets

(11) Veröffentlichungsnummer: **0 131 221 B1**

# (12) EUROPÄISCHE PATENTSCHRIFT

(45) Veröffentlichungstag der Patentschrift: **28.01.87**

(21) Anmeldenummer: **84107548.4**

(22) Anmeldetag: **29.06.84**

(51) Int. Cl.⁴: **C 07 C 149/00**, C 07 C 149/20, C 07 C 149/243, C 07 C 149/24, C 07 C 149/30, C 07 D 295/18, C 07 C 147/14, C 07 C 147/00, A 61 K 31/10

## (54) Thioether, Verfahren zu deren Herstellung sowie diese Verbindungen enthaltende Arzneimittel.

(30) Priorität: **09.07.83 DE 3324916**

(43) Veröffentlichungstag der Anmeldung: **16.01.85 Patentblatt 85/3**

(45) Bekanntmachung des Hinweises auf die Patenterteilung: **28.01.87 Patentblatt 87/5**

(84) Benannte Vertragsstaaten: **AT BE CH DE FR GB IT LI LU NL SE**

(56) Entgegenhaltungen:
**DE - A - 2 130 923**
**FR - A - 2 367 059**
**GB - A - 2 029 977**

**JOURNAL OF THE AMERICAN CHEMICAL SOCIETY, vol. 70, September 1948, Easton, Q.F. SOPER et al.: "Biosynthesis of Penicillins VII. Oxy- and Mercaptoacetic Acids", Seiten 2849-2852**
**JOURNAL OF THE AMERICAN CHEMICAL SOCIETY, vol. 102, 1980, Easton, R.J. PALMER et al.: "Elimination and addition reactions. 36. Acceleration of nucleophilic eliminative ring fission by bond strain", Seiten 7888-7892**

(73) Patentinhaber: **Boehringer Mannheim GmbH, Sandhofer Strasse 116, D-6800 Mannheim 31 (DE)**

(72) Erfinder: **Ummlnger, Robert, Dr. rer. nat., Seckacher Strasse 22, D-6800 Mannheim 52 (DE)**
Erfinder: **Friebe, Walter-Gunnar, Dr. rer. nat., Heidelberger Landstrasse 111d, D-6100 Darmstadt 13 (DE)**
Erfinder: **Kampe, Wolfgang, Dr. rer. nat., Zedernstrasse 49, D-6805 Heddesheim (DE)**
Erfinder: **Roesch, Androniki, Dr. med., Werderstrasse 44, D-6800 Mannheim 1 (DE)**
Erfinder: **Wilhelms, Otto-Henning, Dr. rer. nat., Odenwaldstrasse 25/2, D-6941 Weinheim-Rittenweier (DE)**

EP 0 131 221 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Thioether, Verfahren zu deren Herstellung sowie diese Verbindungen enthaltende Arzneimittel.

Die erfindungsgemässen Verbindungen können bei oraler und bei parenteraler Gabe anaphylaktische und anaphylaktoide Reaktionen hemmen, wie sie beispielsweise an sensibilisierten Meerschweinchen durch Allergenprovokation ausgelöst werden können. Ausserdem wirken sie entzündungshemmend. Sie eignen sich daher zur Bekämpfung von allergischen Krankheiten, beispielsweise von allergischem Asthma.

Bekannt ist aus J. Amer. Chem. Soc. 70, 2849 (1948) die 2-Phenyloxyethylmercaptoessigsäure als Zwischenprodukt zur Herstellung von Penicillinen. In J. Amer. Chem. Soc. 102, 7891 (1980) ist die 3-Phenoxypropylmercaptoessigsäure ohne Angabe einer Verwendung beschrieben.

In der FR-A-2 367 059 sind Verbindungen der Formel I als Schmiermittel beschrieben, in der Ar ein alkylsubstituierter Phenylrest, Q Sauerstoff, A eine $-CH_2-CH(OH)-CH_2$-Gruppe und R Wasserstoff bedeutet.

Die vorliegende Erfindung betrifft Thioether der allgemeinen Formel I

$$\text{Ar-Q-A-}\overset{(O)_n}{\overset{\uparrow}{S}}\text{-R} \qquad (I),$$

in welcher

Ar einen Phenylrest, der ein- bis dreifach durch Alkyl, Alkenyl, Alkoxy, Alkylthio, Alkanoyl, Hydroxyl, Hydroxyalkyl, Carboxyl, Alkoxycarbonyl, Carbamoyl, Nitro, Amino oder Halogen substituiert ist,

Q ein Sauerstoffatom, ein Schwefelatom oder eine NH-Gruppe,

A einen geradkettigen oder verzweigten $C_{2-8}$-Alkylenrest, der gegebenenfalls durch eine Hydroxylgruppe substituiert oder durch eine Ethenylen- oder eine Ethinylen-Gruppe unterbrochen sein kann,

n die Zahl 0, 1 oder 2 und

R einen geradkettigen oder verzweigten Alkylrest, der gegebenenfalls durch einen oder mehrere gleiche oder verschiedene Reste bestehend aus: Alkoxycarbonyl, Alkoxy, Hydroxyl, Carboxyl oder gewünschtenfalls substituiertes Carbamoyl oder Amino substituiert sein kann,

bedeuten,

sowie deren pharmakologisch verträgliche Salze. Weiterhin betrifft die Erfindung pharmazeutische Präparate mit einem Gehalt an Verbindungen der allgemeinen Formel I sowie die Verwendung von Verbindungen der allgemeinen Formel I zur Herstellung solcher Präparate.

Die Alkylreste in sämtlichen genannten Gruppen von Ar und R, allein oder als Bestandteil von Alkoxy-, Alkylthio-, Alkanoyl-, Alkoxycarbonyl- oder Hydroxyalkyl-Resten, enthalten jeweils 1 bis 12, vorzugsweise 1 bis 4 Kohlenstoffatome und können geradkettig oder verzweigt sein. Bevorzugt sind Reste wie Methyl, Ethyl, Propyl oder Pentyl, wie Methoxy oder Ethoxy, wie Methylmercapto, wie Acetyl oder Propionyl, wie Carbmethoxy- oder Carbethoxy, und wie Hydroxymethyl oder 2-Hydroxymethyl. Der Alkylenrest enthält 2 bis 6 Kohlenstoffatome, bevorzugt ist der Allylrest.

Als Halogenatom kommen Fluor, Chlor und Brom in Frage.

Eine substituierte Carbamoyl-Gruppe des Substituenten R bedeutet vorzugsweise eine 2-(4-Morpholino)-ethylaminocarbonyl- oder eine 2-(Dimethylamino)-ethylaminocarbonyl-Gruppe. Eine substituierte Amino-Gruppe des Substituenten R bedeutet vorzugsweise eine Trifluoracetylamino- oder Dimethylamino-Gruppe.

Insbesondere stellt der Substituent R Reste folgender Bedeutung dar:

$-CH_2-COOH$, $-CH_2-CH_2$, $-COOH$,
$-CH_2-CH(CH_3)-COOH$, $-(CH_2)_5-COOH$,
$(CH_2)_{12}-COOH$, $-CH_2-CH(NH_2)-COOH$, $-CH(COOH)-CH_2-COOH$, $-CH_2-CH_2OH$, $-CH_2-CH(OH)-CH_2OH$,

$-CH(CH_3)-CH_2-CH_3$, $-CH_2-CO-NH-CH_2CH_2-$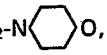,

$-CH_2CH_2-CO-NH-CH_2CH_2-N(CH_3)_2$, $-CH_2CH_2-N(CH_3)_2$.

Die Gruppe A bedeutet vorzugsweise:

$-CH_2-CH_2-$, $CH_2-CH_2-CH_2-$, $-CH_2-CH(OH)-CH_2-$,
$-CH_2-CH_2-CH_2-CH_2-$, $-CH_2-CH=CH-CH_2-$,
$-CH_2-C\equiv C-CH_2-$.

Besonders bevorzugt sind Verbindungen mit der angegebenen Bedeutung von Ar, A und R, bei dem Q = Sauerstoff und n = 0 ist. Ausser den in den Beispielen genannten Verbindungen sind Gegenstand der vorliegenden Erfindung insbesondere alle Substanzen, die jede mögliche Kombination der in den Beispielen genannten Substanzen aufweisen.

Die erfindungsgemässen Verfahren zur Herstellung der Verbindungen der Formel I sind dadurch gekennzeichnet, dass man in an sich bekannter Weise entweder

a) eine Verbindung der allgemeinen Formel II

$$\text{Ar-Q-H} \qquad (II),$$

in der

Ar und Q die oben aufgeführte Bedeutung haben, mit einer Verbindung der allgemeinen Formel III

$$\text{X-A-Y} \qquad (III),$$

in der

A die oben genannte Bedeutung hat und

X und Y reaktive Reste bedeuten,

wobei für den Fall, dass A für die Gruppe $-CH_2CH(OH)CH_2-$ steht, X-A-Y auch 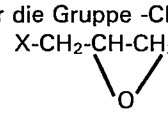 sein kann,

und einer Verbindung der allgemeinen Formel IV

$$\text{R-S-H} \qquad (IV),$$

in der

R die oben genannte Bedeutung hat,

umsetzt

oder

b) eine Verbindung der allgemeinen Formel V

Ar-Q-A-SH (V),

in der

Ar, Q und A die oben aufgeführte Bedeutung haben, mit einer Verbindung der allgemeinen Formel VI

R-X (VI),

in der

R und X die oben genannte Bedeutung haben, zur Reaktion bringt,

anschliessend gewünschtenfalls durch bekannte Verfahren den Schwefel oxidiert

oder den Rest R umwandelt, beispielsweise für den Fall, dass R durch Alkoxycarbonyl substituiert ist, gegebenenfalls zu Carboxyl verseift,

für den Fall, dass R eine geschützte Aminofunktion enthält,

gegebenenfalls die Schutzgruppe abspaltet,

für den Fall, dass R Alkoxy-substituiert ist,

gegebenenfalls in Hydroxy überführt,

und das so erhaltene Reaktionsprodukt gewünschtenfalls in ein pharmakologisch verträgliches Salz überführt.

Als reaktive Reste X und Y der Verbindungen der allgemeinen Formel III kommen Chlor, Brom, Mesyloxy und Tosyloxy in Frage.

Die erfindungsgemässen Verfahren führt man beispielsweise so durch, dass man zunächst eine Verbindung der allgemeinen Formel II mit einer Verbindung der allgemeinen Formel III umsetzt und das erhaltene Reaktionsprodukt isoliert. Dieses Zwischenprodukt wird dann mit einer Verbindung der allgemeinen Formel IV zur Reaktion gebracht. Die Umsetzung erfolgt zweckmässig in neutralem oder in basischem Medium, beispielsweise in einem niederen Alkohol, wie Methanol, mit oder ohne Zusatz von Triethylamin oder in einem niederen Alkohol wie Ethanol, in Gegenwart von einem Alkalialkoholat.

Eine andere Variante besteht darin, dass man zunächst eine Verbindung der Formel IV mit einer Verbindung der Formel III alkyliert und das erhaltene Zwischenprodukt anschliessend mit einer Verbindung der Formel II zur Reaktion bringt.

Ein weiteres Verfahren besteht darin, zunächst eine Verbindung der allgemeinen Formel V, die durch Umsetzung einer Verbindung der Formel II mit einer Verbindung der Formel III und anschliessende Umwandlung der verbliebenen Gruppe X oder Y in eine -SH-Gruppe erhalten werden kann, mit einer Verbindung der allgemeinen Formel VI zur Reaktion bringt. Die Umsetzung erfolgt zweckmässig in neutralem oder basischem Medium, beispielsweise in einem niederen Alkohol, wie Methanol, mit oder ohne Zusatz von Triethylamin oder in einem niederen Alkohol, wie z.B. Ethanol, in Gegenwart von einem Alkalialkoholat.

Eine nachträgliche Umwandlung von Verbindungen der allgemeinen Formel I kann beispielsweise dadurch erfolgen, dass man nach allgemein bekannten Methoden das Sulfid zum Sulfoxid oder zum Sulfon oxidiert.

Eine weitere Möglichkeit, Verbindungen der allgemeinen Formel I nachträglich umzuwandeln, besteht darin, dass man einen oder mehrere Substituenten der Gruppe R in der allgemeinen Formel I durch Veresterung, Verseifung, Reduktion, Alkylierung, Acylierung, Hydrogenolyse, Oxidation, Amidierung oder Eliminierung in einen oder mehrere andere Substituenten des Restes R umwandelt.

Die Ausgangsverbindungen II, III, IV und VI sind literaturbekannte Substanzen oder können in Analogie zu literaturbekannten Verfahren hergestellt werden.

Als pharmakologisch verträgliche Salze kommen insbesondere Alkali-, Erdalkali- und Ammoniumsalze sowie gegebenenfalls Salze mit nicht toxischen anorganischen oder organischen Säuren, wie z.B. Salzsäure, Schwefelsäure, Phosphorsäure, Bromwasserstoffsäure, Essigsäure, Milchsäure, Zitronensäure, Äpfelsäure, Benzoesäure, Salicylsäure, Malonsäure, Maleinsäure, Bernsteinsäure oder Diaminocapronsäure in Frage.

Die Salze erhält man in üblicher Weise z.B. durch Neutralisation der Verbindungen der Formel I mit den entsprechenden Laugen oder Säuren.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Drivate von Sorbitanhydriden.

Feste Trägerstoffe sind z.B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süssstoffe enthalten. Für die äusserliche Anwendung können die erfindungsgemässen Substanzen I auch in Form von Pudern und Salben verwendet werden. Sie werden dazu z.B. mit pulverförmigen, physiologisch veträglichen Verdünnungsmitteln bzw. üblichen Salbengrundlagen vermischt.

Die verabreichte Dosis hängt vom Alter, der Gesundheit und dem Gewicht des Empfängers, dem Ausmass der Krankheit, der Art gleichzeitiger gegebenenfalls durchgeführter weiterer Behandlungen,

der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg/Tag in einer oder mehreren Anwendungen pro Tag wirksam, um die gewünschten Resultate zu erhalten.

*Beispiel 1*

*S-[4-(4-Acetyl-3-hydroxy-2-propyl-phenoxy)butyl]-3-mercaptopropionsäure*

Eine Mischung aus 5,5 g (17 mmol) 4-(4-Acetyl-3-hydroxy-2-propyl-phenoxy)-n-butylbromid, 1,9 g (18 mmol) 3-Mercaptopropionsäure, 7,25 g (70 mmol) Triethylamin und 25 ml Methanol wird vier Stunden lang unter Rückfluss erhitzt. Nach dem Abkühlen versetzt man mit Wasser, säuert an und extrahiert mit Dichlormethan. Die organische Phase wird neutral gewaschen, getrocknet und eingeengt. Der Rückstand wird in Dichlormethan aufgenommen und an Kieselgel chromatographiert (Elutionsmittel: Dichlormethan/Methanol 98 : 2).

Man erhält nach dem Anreiben mit Diethylether 2,8 g (46% d.Th.) der Titelverbindung vom Schmp. 75-77°C.

*Beispiel 2*

In analoger Weise wie in Beispiel 1 beschrieben erhält man:

| | Bezeichnung | Ausbeute % | Schmelzpunkt °C (Lösungsmittel) |
|---|---|---|---|
| a) | S-[3-(3-Chlor-phenoxy)propyl]-3-mercaptopropionsäure aus 3-(3-Chlor-phenoxy)propylbromid und 3-Mercapto-propionsäure | 69 | 40-42 (Dichlormethan) |
| b) | S-[3-(3-Nitro-phenoxy)propyl]-3-mercapto-propionsäure aus 3-(3-Nitro-phenoxy)propylbromid und 3-Mercapto-propionsäure | 44 | 58-61 (Diethylether) |
| c) | S-[3-(2-Pentyl-phenoxy)propyl]-3-mercapto-propionsäure aus 3-(2-Pentyl-phenoxy)propylbromid und 3-Mercapto-propionsäure | 31 | 35-37 (Dichlormethan) |
| d) | S-[3-(4-Acetyl-3-hydroxy-phenoxy)-propyl]-2-mercapto-ethanol aus 3-(4-Acetyl-3-hydroxy-phenoxy)-propylbromid und 2-Mercapto-ethanol | 82 | 50-52 (Diethylether) |
| e) | S-[3-(4-Acetyl-3-hydroxy-phenoxy)-propyl]-3-mercapto-propionsäure aus 3-(4-Acetyl-3-hydroxy-phenoxy)-propylbromid und 3-Mercapto-propionsäure | 58 | 116-119 amorph (Diethylether) |
| f) | S-[3-(4-Acetyl-2-allyl-3-hydroxy-phenoxy)propyl]-2-mercapto-ethanol aus 3-(4-Acetyl-2-allyl-3-hydroxy-phenoxy)propylbromid und 2-Mercapto-ethanol | 51 | 61-63 (Methanol) |
| g | S-[3-(4-Acetyl-2-allyl-3-hydroxy-phenoxy)propyl]-2-hydroxy-3-mercapto-propanol aus 3-(4-Acetyl-2-allyl-3-hydroxy-phenoxy)propylbromid und 2-Hydroxy-3-mercapto-propanol | 84 | 58-60 (Diethylether) |
| h) | S-[3-(4-Acetyl-3-hydroxy-2-propyl-phenoxy)propyl]-3-mercapto-propionsäure aus 3-(4-Acetyl-3-hydroxy-2-propyl-phenoxy)propylbromid und 3-Mercapto-propionsäure | 75 | 90-93 (Diethylether) |
| i) | S-[3-(4-Acetyl-2-allyl-3-hydroxy-phenoxy)propyl]-3-mercapto-propionsäure aus 3-(4-Acetyl-2-allyl-3-hydroxy-phenoxy)propylbromid und 3-Mercapto-propionsäure | 53 | 96-99 (Methanol) |

*Beispiel 2* (Fortsetzung)

| | Bezeichnung | Ausbeute % | Schmelzpunkt °C (Lösungsmittel) |
|---|---|---|---|
| j) | S-[3-(4-Acetyl-3-hydroxy-2-propyl-phenoxy)propyl]-DL-mercapto-bernsteinsäure aus 3-(4-Acetyl-3-hydroxy-2-propyl-phenoxy)propylbromid und DL-Mercapto-bernsteinsäure | 47 | 126-128 (Methanol/Wasser) |
| k) | S-[4-(4-Acetyl-3-hydroxy-2-propyl-phenoxy)-n-butyl]-2-mercapto-ethanol aus 4-(4-Acetyl-3-hydroxy-2-propyl-phenoxy)-n-butylbromid und 2-Mercapto-ethanol | 64 | Öl |
| l) | S-[3-(4-Methylmercapto-phenoxy)-propyl]-3-mercapto-propionsäure aus 3-(4-Methylmercapto-phenoxy)-propylbromid und 3-Mercapto-propionsäure | 29 | 62-64 (Diethylether) |
| m) | S-[3-(2-(2-Hydroxy-ethyl)phenoxy)-propyl]-3-mercapto-propionsäure aus 3-[2-(2-Hydroxy-ethyl)phenoxy]-propylbromid und 3-Mercapto-propionsäure | 34 | 86-88 (Diethylether/Ligroin) |
| n) | S-[3-(2-Carboxy-phenoxy)propyl]-3-mercapto-propionsäure aus 3-(2-Carboxy-phenoxy)propyl-bromid und 3-Mercapto-propionsäure | 77 | 83-85 (Diethylether) |
| o) | S-[3-(2-Ethoxycarbonyl-phenoxy)-propyl]-3-mercapto-propionsäure aus 3-(2-Ethoxycarbonyl-phenoxy)-propylbromid und 3-Mercapto-propionsäure | 51 | 46-49 (Diethylether/Ligroin) |
| p) | S-[3-(2-Carbamoyl-phenoxy)propyl]-3-mercapto-propionsäure aus 3-(2-Carbamoyl-phenoxy)propyl-bromid und 3-Mercapto-propionsäure | 57 | 114-116 (Essigsäureethylester) |
| q) | S-[3-(3-Pentyl-phenoxy)propyl]-3-mercapto-propionsäure aus 3-(3-Pentyl-phenoxy)propylbromid und 3-Mercapto-propionsäure | 38 | Na-Salz 176-181 amorph |
| r) | 2-〈3-[S-(2-Butyl)mercapto]propoxy〉-benzoesäure aus 2-(3-Brom-propoxy)benzoesäure und 2-Butylmercaptan | 52 | Na-Salz 271 Zers. amorph |
| s) | S-[3-(4-Acetyl-3-hydroxy-2-propyl-phenoxy)propy]-3-mercapto-2-methyl-propionsäure aus 3-(4-Acetyl-3-hydroxy-2-propyl-phenoxy)propyl-bromid und 3-Mercapto-2-methyl-propionsäure | 41 | 90-93 Zers. Na-Salz amorph. |

*Beispiel 3*

*S-[3-(3-Methoxy-phenoxy)propyl]-3-mercapto-propionsäure*

Zu einer Lösung von 1,15 g (50 mmol) Natrium in 50 ml Ethanol gibt man bei Raumtemperatur 2,65 g (25 mmol) 3-Mercaptopropionsäure, rührt zehn Minuten lang bei Raumtemperatur, fügt 6,12 g (25 mmol) 3-(3-Methoxy-phenoxy)-propylbromid, gelöst in 10 ml Ethanol, zu, erhitzt zwei Stunden lang unter Rückfluss und destilliert dann das Lösungsmittel ab. Man nimmt den Rückstand in Wasser auf, säuert an und extrahiert mit Diethylether.

Der nach dem Trocknen und Einengen der Extrakte erhaltene Rückstand liefert beim Anreiben mit Diethylether 5,5 g (82% d.Th.) Titelverbindung vom Schmp. 53-55°C.

*Beispiel 4*

In analoger Weise wie in Beispiel 3 beschrieben erhält man:

| | Bezeichnung | Ausbeute % | Schmelzpunkt °C (Lösungsmittel) |
|---|---|---|---|
| a) | S-[3-(4-Acetyl-phenoxy)propyl]-3-mercapto-propionsäure aus 3-(4-Acetyl-phenoxy)propylbromid und 3-Mercapto-propionsäure | 80 | 125-126 ($H_2O$) |
| b) | S-[3-(2-Propyl-phenoxy)propyl]-3-mercapto-propionsäure aus 3-(2-Propyl-phenoxy)propyl-bromid und 3-Mercapto-propionsäure | 55 | $Kp_{0,4}$ 200-202 |
| c) | S-[3-(4-Acetyl-2-propyl-phenoxy)-propyl]-3-mercapto-propionsäure aus 3-(4-Acetyl-2-propyl-phenoxy)-propylbromid und 3 -Mercapto-propionsäure | 61 | 42-45 (Wasser) |
| d) | S-[3-(3-Methoxy-5-methyl-phenoxy)-propyl]-3-mercapto-propionsäure aus 3-(3-Methoxy-5-methyl-phenoxy)-propylbromid und 3-Mercapto-propionsäure | 52 | 38-40 (Ligroin) |
| e) | S-[3-(3,5-Dimethoxy-phenoxy)propyl]-3-mercapto-propionsäure aus 3-(3,5-Dimethoxy-phenoxy)propyl-bromid und 3-Mercapto--propionsäure | 70 | 50-52 |
| f) | S-[3-(4-Acetyl-3-hydroxy-2-propyl-phenoxy)propyl]-L-cystein aus 3-(4-Acetyl-3-hydroxy-2-propyl-phenoxy)propylbromid und L-Cystein | 40 | 155-160 ($H_2O$) |
| g) | S-[4-(4-Acetyl-3-hydroxy-2-propyl-phenoxy)-n-butyl]-L-cystein aus 4-(4-Acetyl-3-hydroxy-2-propyl-phenoxy)-n-butylbromid und L-Cystein | 50 | Na-Salz 110-120 amorph ($H_2O$) |
| h) | S-[3-(3-Hydroxy-phenoxy)propyl]-3- mercapto-propionsäure aus 3-(3-Hydroxy-phenoxy)propyl-bromid und 3-Mercapto-propionsäure | 41 | $Kp_{0,5}$ 208-215 |
| i) | S-[3-(4-Acetyl-3-hydroxy-2-propyl-phenoxy)propyl]-2-mercapto--ethanol aus 3-(4-Acetyl-3-hydroxy-2-propyl-phenoxy)propyl-bromid und Mercaptoethanol | 94 | 72-73 (Diethylether) |

*Beispiel 5*

*S-[4-(4-Acetyl-3-hydroxy-2-propyl-phenoxy)-2--butinyl]-2-mercapto-ethanol*

1,41 g (5 mmol) 4-(4-Acetyl-3-hydroxy-2-propyl-phenoxy)-2-butinylchlorid werden zusammen mit 0,38 g (4,9 mmol) 2-Mercapto-ethanol, 3 g (30 mmol) Triethylamin und 5 ml Methanol fünf Stunden lang unter Rückfluss gekockt. Man lässt abkühlen, versetzt mit Wasser, säuert an und extrahiert mit Dichlormethan. Die vereinigten Extrakte werden neutral gewaschen, getrocknet und eingeengt. Zur Reinigung des Rohproduktes wird an Kieselgel chromatographiert (Elutionsmittel: Dichlormethan/Methanol 98 : 2).

Durch Anreiben mit Diethylether erhält man 1,1 g (68% d.Th.) kristallines Produkt von Schmp. 52-53°C.

*Beispiel 6*

In analoger Weise wie in Beispiel 5 beschrieben erhält man:

| | Bezeichnung | Ausbeute % | Schmelzpunkt °C (Lösungsmittel) |
|---|---|---|---|
| a) | S-[4-(4-Acetyl-3-hydroxy-2-propyl-phenoxy)-2-butenyl]-2-mercapto-ethanol aus 4-(4-Acetyl-3-hydroxy-2-propyl-phenoxy)-2-butenylchlorid und 2-Mercapto-ethanol | 63 | Öl |
| b) | S-[4-(4-Acetyl-3-hydroxy-2-propyl-phenoxy)-2-butenyl]mercapto--essigsäure aus 4-(4-Acetyl-3-hydroxy-2-propyl-phenoxy)-2-butenylchlorid und Mercapto-essigsäure | 52 | 66-68 (Ligroin) |
| c) | S-[4-(4-Acetyl-3-hydroxy-2-propyl-phenoxy)-2-butenyl]-3-mercapto-propionsäure aus 4-(4-Acetyl-e-3hydroxy-2-propyl-phenoxy)-2-butenylchlorid und 3-mercapto-propionsäure | 55 | 89-91 (Diethylether) |
| d) | S-[4-(4-Acetyl-3-hydroxy-2-propyl-phenoxy)-2-butinyl]mercapto--essigsäure aus 4-(4-Acetyl-3-hydroxy-2-propyl-phenoxy)-2-butinylchlorid und Mercapto-essigsäure | 79 | 111-112 (Ligroin) |
| e) | S-[4-(4-Acetyl-3-hydroxy-2-propyl-phenoxy)-2-butinyl]-3-mercapto-propionsäure aus 4-(4-Acetyl-3-hydroxy-2-propyl-phenoxy)-2-butinylchlorid und 3-Mercapto-propionsäure | 69 | 88-90 (Diethylether) |

*Beispiel 7*

*S-[3-(4-Acetyl-2-allyl-3-hydroxy-phenoxy)-2-hydroxy-propyl]-2-mercapto-ethanol*

Zu einer Lösung von 7,45 g (30 mmol) 3-(4-Acetyl-2-allyl-3-hydroxy-phenoxy)-1,2-epoxypropan in 35 ml Methanol und 12,1 g (120 mmol) Triethylamin gibt man 2,58 g (33 mmol) 2-Mercapto-ethanol und rührt drei Stunden lang bei Raumtemperatur. Danach wird das Lösungsmittel abdestilliert, der Rückstand in Dichlormethan aufgenommen, eingeengt, neutral gewaschen und an Kieselgel chromatographiert (Elutionsmittel: Dichlormethan/Methanol 9 : 1).

Nach Anreiben mit Diethylether erhält man 4,5 g (46% d.Th.) der Titelverbindung vom Schmp. 54-56°C.

*Beispiel 8*

In analoger Weise wie in Beispiel 7 beschrieben erhält man:

| | Bezeichnung | Ausbeute % | Schmelzpunkt °C (Lösungsmittel) |
|---|---|---|---|
| a) | S-[3-(4-Acetyl-2-allyl-3-hydroxy-phenoxy)2-hydroxy-propyl]--2-hydroxy-3-mercapto-propanol aus 3-(4-Acetyl-2-allyl-3-hydroxy-phenoxy)-1,2-epoxy-propan und 2-Hydroxy-3-mercapto-propanol | 40 | Öl |
| b) | S-[3-(4-Acetyl-3-hydroxy-2-propyl-phenoxy)-2-hydroxy-propyl]--2-mercapto-ethanol aus S-[3-(4-Acetyl-3-hydroxy-2-propyl-phenoxy)-1,2-epoxy--propan und 2-Mercapto-ethanol | 64 | 56-57 (Diethylether) |

*Beispiel 9*

*S-[3-(4-Acetyl-2-allyl-3 hydroxy-phenoxy]-2-hydroxy-propyl]-3-mercapto-propionsäure*

Zu einer Lösung von 7,5 g (30 mmol) 3-(4-Acetyl-2-allyl-3-hydroxy-phenoxy)-1,2-epoxy-propan in 35 ml Methanol und 12,1 g (120 mmol) Triethylamin gibt man 3,5 g (33 mmol) 3-Mercaptopropionsäure und rührt zehn Stunden lang bei Raumtemperatur. Danach wird eingeengt, der Rückstand in Wasser aufgenommen, angesäuert und mit Dichlormethan extrahiert. Nach dem Trocknen und Einengen der Lösung erhält man 7,2 g (68% d.Th.) der Titelverbindung als Öl.

Versetzt man eine ethanolische Lösung des erhaltenen Produktes mit wässriger L-Lysinlösung (Molverhältnis 1 : 1), wird die Titelverbindung quantitativ als Lysinsalz gefällt. Schmp. 96-99°C.

*Beispiel 10*

In analoger Weise wie in Beispiel 9 beschriebenen erhält man:

| | Bezeichnung | Ausbeute % | Schmelzpunkt °C (Lösungsmittel) |
|---|---|---|---|
| a) | S-[3-(4-Acetyl-3-hydroxy-2-propyl-phenoxy)-2-hydroxy-propyl]-L-cystein aus 3-(4-Acetyl-3-hydroxy-2-propyl-phenoxy)-1,2-epoxy-propan und L-Cystein | 51 | 141-142 (Dichlormethan) |
| b) | S-[3-(4-Acetyl-2-allyl-3-hydroxy-phenoxy)-2-hydroxy-propyl]-L-cystein aus 3-(4-Acetyl-2-allyl-3-hydroxy-phenoxy)-1,2-epoxy-propan und L-Cystein | 60 | 159-161 Zers. (Dichlormethan) |
| c) | S-[3-(4-Acetyl-2-allyl-3-hydroxy-phenoxy)-2-hydroxy-propyl]-N-Trifluoracetyl-L-cystein aus 3-(4-Acetyl-2-allyl-3-hydroxy-phenoxy)-1,2-epoxy-propan und N-Trifluoracetyl-L-cystein | 56 | 75 amorph |
| d) | S-[3-(3-Chlor-phenoxy)-2-hydroxy-propyl]-3-mercapto-propionsäure aus 3-(3-Chlor-phenoxy)-1,2-epoxy-propan und 3-Mercapto-propionsäure | 31 | 58-60 (Dichlormethan) |
| e) | S-[3-(3-Chlor-phenoxy)-2-hydroxy-propyl]-cystein aus 3-(3-Chlor-phenoxy)-1,2-epoxy-propan und L-Cystein | 68 | 176-180 (Diethylether) |
| f) | S-[2-Hydroxy-3-(2-propyl-phenoxy)propyl]-3-mercapto-propionsäure aus 3-(2-Propyl-phenoxy)-1,2-epoxy-propan und 3-Mercapto-propionsäure | 78 | Na-Salz 60-70 amorph (Wasser) |
| g) | S-[2-Hydroxy-3-(2-propyl-phenoxy)propyl]-cystein aus 3-(2-Propyl-phenoxy)-1,2-epoxy-propan und L-Cystein | 76 | 178-181 (Aceton) |
| h) | S-[2-Hydroxy-3-(3-methoxy-phenoxy)propyl]-3-mercapto-propionsäure aus 3-(3-Methoxy-phenoxy)-1,2-epoxy-propan und 3-Mercapto-propionsäure | 75 | Na-Salz 40-50 amorph (Wasser) |
| i) | S-[2-Hydroxy-3-(3-methoxy-phenoxy)propyl]-cystein aus 3-(3-Methoxy-phenoxy)-1,2-epoxy-propan und L-Cystein | 62 | 182 (Wasser) |
| j) | S-[2-Hydroxy-3-(2-pentyl-phenoxy)propyl]-3-mercapto-propionsäure aus 3-(2-Pentyl-phenoxy)-1,2-epoxy-propan und 3-Mercapto-propionsäure | 64 | Na-Salz 92-96 amorph |

*Beispiel 10* (Fortsetzung)

| | Bezeichnung | Ausbeute % | Schmelzpunkt °C (Lösungsmittel) |
|---|---|---|---|
| k) | S-(2-Hydroxy-3-(2-pentyl-phenoxy)propyl]-cystein aus 3-(2-Pentyl-phenoxy)-1,2-epoxy-propan und L-Cystein | 71 | HCl-Salz 150-152 (Wasser) |
| l) | S-[3-(4-Acetyl-3-hydroxy-2-propyl-phenoxy)-2-hydroxy-propyl]-3-mercapto-propionsäure aus 3-(4-Acetyl-3-hydroxy-2-propyl-phenoxy)-1,2-epoxy-propan und 3-Mercapto-propionsäure | 73 | 104-107 (Dichlormethan) |

*Beispiel 11*

*S-[3-(4-Acetyl-3-hydroxy-2-propyl-phenoxy)-propyl]-3-mercapto-propionsäure*

1,34 g (5 mmol) 3-(4-Acetyl-3-hydroxy-2-propyl-phenoxy)-propyl-mercaptan, 0,9 g (6 mmol) 3-Brompropionsäure und 2,8 ml (20 mmol) Triethylamin werden in 5 ml Methanol vier Stunden lang unter Rückfluss erhitzt. Nach dem Abkühlen versetzt man mit Wasser, säuert an und extrahiert mit Dichlormethan. Die organische Phase wird neutral gewaschen, getrocknet und eingeengt. Das erhaltene Öl wird an Kieselgel chromatographiert (Elutionsmittel: Dichlormethan mit steigendem Anteil an Methanol, maximales Mischungsverhältnis 95 : 5).

Nach Anreiben mit Diethylether erhält man 0,9 g (47% d.Th.) der Titelverbindung von Schmp. 90-92°C.

*Beispiel 12*

In analoger Weise wie in Beispiel 11 beschrieben erhält man:

| | Bezeichnung | Ausbeute % | Schmelzpunkt °C (Lösungsmittel) |
|---|---|---|---|
| a) | S-[3-(4-Acetyl-3-hydroxy-2-propyl-phenoxy)-propyl]-6-mercapto-capronsäure aus 3-[4-Acetyl-3-hydroxy-2-propyl-phenoxy)propyl-mercaptan (Beisp. 12) und 6-Brom-capronsäure | 50 | Na-Salz 82-84 amorph (Wasser) |
| b) | S-[3-(4-Acetyl-3-hydroxy-2-propyl-phenoxy)-propyl]-12-mercapto-dodecansäure aus 3-(4-Acetyl-3-hydroxy-2-propyl-phenoxy)-propyl-mercaptan (Beisp. 12) und 12-Brom-dodecansäure | 44 | 63-65 Ligroin |

*Beispiel 13*

*S-[3-(3-Methoxy-5-methyl-phenoxy)propyl]-3-mercapto-propionsäure*

Zu einer Suspension von 2,2 g (44 mmol) 50 proz. Natriumhydrid in 50 ml Dimethylformamid tropft man die Lösung von 3,0 g (22 mmol) 3-Methoxy-5-methyl-phenol in 10 ml Dimethylformamid, rührt 10 min bei Raumtemperatur, tropft die Lösung von 3,15 g (22 mmol) S-(3-Chlor-propyl)-3-mercapto-propionsäure in 10 ml Dimethylformamid zu und erhitzt 4 Stunden auf 100°C. Darauf wird im Vakuum eingeengt, mit Ether versetzt, mit Wasser extrahiert, die wässrige Phase angesäuert und mit Ether extrahiert.

Nach Einengen verbleiben 2,9 g Titelverbindung (46% d.Th.) vom Schmp. 39-42°C (aus Ligroin ).

*Beispiel 14*

In analoger Weise wie in Beispiel 13 beschrieben erhält man:

| Bezeichnung | Ausbeute % | Schmelzpunkt °C (Lösungsmittel) |
|---|---|---|
| S-[3-(3-Hydroxy-2-propyl-phenoxy)propyl]-3-mercapto-propionsäure aus 2-Propyl-resorcin und S-(3-Chlor-propyl)-3-mercapto-propionsäure | 30 | Na-Salz 40-50 amorph ($H_2O$) |

*Beispiel 15*

*3-[3-(4-Acetyl-2-allyl-3-hydroxy-phenoxy)propyl]-sulfinyl-propionsäure*

Man versetzt 1,7 g (5 mmol) S-[3-(4-Acetyl-2-allyl-3-hydroxy-phenoxy)propyl]-3-mercapto-propionsäure mit einer Lösung von 0,55 ml (5 mmol) Wasserstoffperoxid in 20 ml Eisessig und rührt solange bei Raumtemperatur, bis dünnschichtchromatographisch kein Ausgangsmaterial mehr nachzuweisen ist. Danach wird das Lösungsmittel im Vakuum abdestilliert und der Rückstand aus Aceton umkristallisiert.

Man erhält 1 g (56% d.TH) Titelverbindung vom Schmp. 126-128°C.

*Beispiel 15*

In analoger Weise wie in Beiepiel 15 beschrieben erhält man:

| | Bezeichnung | Ausbeute % | Schmelzpunkt °C (Lösungsmittel) |
|---|---|---|---|
| a) | 3-[3-(4-Acetyl-3-hydroxy-2-propyl-phenoxy)propyl]sulfinyl-propionsäure aus S-[3-(4-Acetyl-2-propyl-3-hydroxy-phenoxy)propyl]-3-mercapto-propionsäure und Wasserstoffperoxid | 87 | 118-121 (Aceton) |
| b) | 2-[3-(4-Acetyl-2-allyl-3-hydroxy-phenoxy)-2-hydroxy-propyl)-sulfinyl-ethanol aus S-[3-(4-Acetyl-2-allyl-3-hydroxy-phenoxy)-2-hydroxy-propyl]-2-mercapto-ethanol (Beisp. 7) und Wasserstoffperoxid | 83 | 142-144 (Dichlormethan) |
| c) | 2-[3-(4-Acetyl-2-allyl-3-hydroxy-phenoxy)propyl]sulfinyl-ethanol aus S-[3-(4-Acetyl-2-allyl-3-hydroxy-phenoxy)propyl]-2-mercapto-ethanol (Beisp. 4 m) und Wasserstoffperoxid | 70 | 95-98 (Dichlormethan) |
| d) | 2-[3-(4-Acetyl-3-hydroxy-2-propyl-phenoxy)propyl]sulfinyl-ethanol aus S-[3-(4-Acetyl-3-hydroxy-2-propyl-phenoxy)propyl]-2-mercapto-ethanol (Beisp. 4 m) und Wasserstoffperoxid | 78 | 73-74 (Dichlormethan) |
| e) | 3-[3-(2-Propyl-phenoxy)propyl]sulfinyl-propionsäure aus S-[3-(2-Propyl-phenoxy)propyl]-3-mercapto-propionsäure (Beisp. 4 d) und Wasserstoffperoxid | 89 | Na-Salz 110-120 amorph (Wasser) |
| f) | 3-[3-(3-Methoxy-phenoxy)propyl]sulfinyl-propionsäure aus S-[3-(3-Methoxy-phenoxy)propyl]-3-mercapto-propionsäure (Beisp. 3) und Wasserstoffperoxid | 63 | Na-Salz 130-140 amorph (Wasser) |
| g) | 2-[3-(4-Acetyl-3-hydroxy-2-propyl-phenoxy)-2-hydroxy-propyl]-sulfinyl-ethanol aus S-[3-(4-Acetyl-3-hydroxy-2-propyl-phenoxy)-2-hydroxy-propyl]-2-mercapto-ethanol (Beisp. 8) und Wasserstoffperoxid | 64 | 55-57 (Diethylether) |
| h) | 3-[3-(4-Acetyl-3-hydroxy-2-propyl-phenoxy)-2-hydroxy-propyl]-sulfinyl-propionsäure aus S-[3-(4-Acetyl-3-hydroxy-2-propyl-phenoxy)-2-hydroxy-propyl]-3-mercapto-propionsäure (Beisp. 10 l) und Wasserstoffperoxid | 73 | 104-107 (Dichlormethan/ Methanol) |
| i) | 3-[3-(4-Acetyl-2-allyl-3-hydroxy-phenoxy)-2-hydroxy-propyl]-sulfinyl-propionsäure aus S-[3-(4-Acetyl-2-allyl-3-hydroxy-phenoxy)-2-hydroxy-propyl]-3-mercapto-propionsäure (Beisp. 9) und Wasserstoffperoxid | 80 | 137-140 (Dichlormethan) |

*Beispiel 17*

*-[3-(4-Acetyl-2-allyl-3-hydroxy-phenoxy)propyl]-sulfonyl-propionsäure*

Man versetzt 1,7 g (5 mmol) S-[3-(4-Acetyl-2--allyl-3-hydroxy-phenoxy7propyl]-3-mercapto-propionsäure mit einer Lösung von 1,63 ml (15 mmol) Wasserstoffperoxid in 20 ml Eisessig und rührt solange bei Raumtemperatur, bis dünnschichtchromatographisch kein Ausgangsmaterial mehr nachzuweisen ist. Der ausgefallene Niederschlag wird abgesaugt und zunächst mit Essigsäureethylester, danach mit Dichlormethan gewaschen.

Man erhält 1,2 g (65% d.Th.) Titelverbindung vom Schmp. 164-166°C.

*Beispiel 18*

In analoger Weise wie in Beispiel 17 beschrieben erhält man:

| | Bezeichnung | Ausbeute % | Schmelzpunkt °C (Lösungsmittel) |
|---|---|---|---|
| a) | 3-[3-(4-Acetyl-3-hydroxy-2-propyl-phenoxy)propyl]sulfonyl--propionsäure aus S-[3-(4-Acetyl-3-hydroxy-2-propyl-phenoxy)propyl]-3--mercapto-propionsäure und Wasserstoffperoxid | 56 | 160-162 (Diethylether) |
| b) | 2-[3-(4-Acetyl-2-allyl-3-hydroxy-phenoxy)-2-hydroxy-propyl]-sulfonyl-ethanol aus S-[3-(4-Acetyl-2-allyl-3-hydroxy-phenoxy)-2-hydroxy--propyl]-2-mercapto-ethanol (Beisp. 7) und Wasserstoffperoxid | 69 | 126-128 |

*Beispiel 19*

*S-[3-(4-Acetyl-3-hydroxy-2-propyl-phenoxy)-2-hydroxy-propyl]-2-mercapto-N,N-dimethyl-ethyl--amin*

Man gibt 7,45 g (30 mmol) 3-(4-Acetyl-2-allyl-3--hydroxy-phenoxy)-1,2-epoxy-propan, gelöst in 30 ml Methanol, 6,3 g (45 mmol) 2-Dimethylamino--ethyl-mercaptan und 13,5 g (135 mmol) Triethylamin zusammen und lässt vier Stunden bei Raumtemperatur rühren. Danach wird das Lösungsmittel abdestilliert, der Rückstand mit Wasser und Essigsäureethylester versetzt, die Extrakte dreimal mit 1 N Natronlauge und einmal mit 1 N Salzsäure ausgeschüttelt, mit 2 N Natronlauge alkalisch gestellt und dreimal mit Essigsäureethylester extrahiert. Waschen, Trocknen und Einengen der Auszüge liefern 8,1 g Öl, das an Kieselgel chromatographiert wird (Eluationsmittel: Dichlormethan, Methanol, Wasser im Verhältnis: 6,5 : 2,5 : 0,4).

Ausbeute: 5,2 g (48% d.Th.) vom Schmp. 54-55°C.

*Beispiel 20*

*3-[3-(4-Acetyl-2-allyl-3-hydroxy-phenoxy)propyl]-sulfinyl-propionsäure-(2-dimethylamino-ethyl)amid*

Zu einer Suspension von 1,06 g (3 mmol) 3-[3-(4--Acetyl-2-allyl-3-hydroxy-phenoxy)propyl]sulfinyl--propionsäure in 20 ml Dichlormethan und 10 ml Tetrahydrofuran gibt man 0,31 g (3 mmol) N-Methylmorpholin, kühlt auf —10°C ab und tropft bei dieser Temperatur 0,43 g (3 mmol) Chlorameisensäure-iso--butylester, gelöst in 5 ml Dichlormethan, zu. Anschliessend wird 15 Minuten lang bei —10°C gerührt und dann 0,26 g (3 mmol) N,N-Dimethyl--ethylendiamin, gelöst in 5 ml Dichlormethan, so zugetropft, dass die Temperatur nicht über —10°C ansteigt. Danach rührt man eine Stunde bei —10°C, zwei Stunden bei °C und lässt dann auf Raumtemperatur erwärmen. Die Reaktionsmischung wird mit Wasser versetzt, mit 2 N Salzsäure sauer gestellt, die wässrige Phase abgetrennt und zweimal mit Dichlormethan extrahiert. Daraufhin stellt man die wässrige Phase mit 2 N Natronlauge alkalisch, extrahiert dreimal mit Dichlormethan, wäscht die Extrakte mit Wasser neutral, trocknet sie und engt ein.

Das Rohprodukt wird in Methanol/Dichlormethan gelöst und die Titelverbindung mit etherischer Salzsäure als HCl-Salz gefällt.

Ausbeute: 0,85 g (67% d.Th.) zerfliessliche Kristalle.

*Beispiel 21*

Es wurden Tabletten hergestellt: Jede Tablette enthält 10 mg 3-[3-(4-Acetyl-2-allyl-3-hydroxy-phenoxy)propyl]-sulfinyl-propionsäure. Die Tabletten wurden gemäss der folgenden Rezeptur hergestellt:

| | |
|---|---|
| 3-[3-(4-Acetyl-2-allyl-3-hydroxy-phenoxy) propyl]sulfonyl-propionsäure | 10 g |
| Lactose | 80 g |
| Stärke | 29 g |
| Magnesiumstearat | 1 g |

Vorstehende Verbindung wurde fein pulverisiert und mit Lactose und Stärke vermischt. Das Gemisch wurde in herkömmlicher Weise granuliert. Magnesiumstearat wurde zu dem Granulat gegeben und das Gemisch zu 1000 Tabletten mit einem Einzelgewicht von 0,12 g verpresst.

**Patentanprüche**

1. Thioesther der allgemeinen Formel I

$$\overset{(O)_n}{\uparrow}$$
$$Ar\text{-}Q\text{-}A\text{-}S\text{-}R \qquad (I),$$

in welcher

Ar einen Phenylrest, der ein- bis dreifach durch Alkyl, Alkenyl, Alkoxy, Alkylthio, Alkanoyl, Hydroxyl, Hydroxyalkyl, Carboxyl, Alkoxycarbonyl, Carbamoyl, Nitro, Amino oder Halogen substituiert ist,

Q ein Sauerstoffatom, ein Schwefelatom oder eine NH-Gruppe,

A einen geradkettigen oder verzweigten $C_{2-8}$-Alkylenrest, der gegebenenfalls durch eine Hydroxylgruppe substituiert oder durch eine Ethenylen- oder eine Ethinylen-Gruppe unterbrochen sein kann,

n die Zahl 0, 1 oder 2 und

R einen geradkettigen oder verzweigten Alkylrest, der gegebenenfalls durch einen oder mehrere gleiche oder verschiedene Reste bestehend aus: Alkoxycarbonyl, Alkoxy, Hydroxy, Carboxyl oder gewünschtenfalls substituiertes Carbamoyl oder Amino substituiert sein kann,

bedeuten,

sowie deren pharmakologisch verträgliche Salze.

2. Thioether gemäss Anspruch 1, in denen Ar einen 4-Acetyl-3-hydroxy-phenyl-, einen 4-Acetyl-2--allyl-3-hydroxy-phenyl-, einen 4-Acetyl-3-hydroxy-2-propyl-phenyl- oder einen 2-Carboxyphenyl-Rest darstellt.

3. Thioether gemäss Anspruch 1 oder 2, in denen Q ein Sauerstoffatom darstellt.

4. Thioether gemäss Anspruch 1, 2 oder 3, in denen A einen $-CH_2-CH_2CH_2-$ oder $-CH_2-CH(OH)-CH_2-$ Rest darstellt.

5. Thioether gemäss Anspruch 1, 2, 3, oder 4, in denen R eine $-CH_2-COOH-$, $-CH_2CH_2-COOH-$, $-CH_2-CH(NH_2)-COOH$ oder eine $-CH_2CH_2-OH$-Gruppe darstellt.

6. Thioether gemäss Anspruch 1, 2, 3, 4, oder 5, in denen n die Zahl 0 darstellt.

7. Verfahren zur Herstellung von Thioethern der allgemeinen Formel I

$$\overset{(O)_n}{\uparrow}$$
$$Ar\text{-}Q\text{-}A\text{-}S\text{-}R \qquad (I),$$

in welcher

Ar einen Phenylrest, der ein- bis dreifach durch Alkyl, Alkenyl, Alkoxy, Alkylthio, Alkanoyl, Hydroxyl, Hydroxyalkyl, Carboxyl, Alkoxyxcarbonyl, Carbamoyl, Nitro, Amino oder Halogen substituiert ist,

Q ein Sauerstoffatom, ein Schwefelatom oder eine NH-Gruppe,

A einen geradkettigen oder verzweigten $C_{2-8}$-Alkylenrest, der gegebenenfalls durch eine Hydroxylgruppe substituiert oder durch eine Ethenylen- oder eine Ethinylen-Gruppe unterbrochen sein kann,

n die Zahl 0, 1 oder 2 und

R einen geradkettigen oder verzweigten Alkylrest, der gegebenenfalls durch einen oder mehrere gleiche oder verschiedene Reste bestehend aus: Alkoxycarbonyl, Alkoxy, Hydroxyl, Carboxyl oder gewünschtenfalls substituiertes Carbamoyl oder Amino substituiert sein kann,

bedeuten,

sowie deren pharmakologisch veträgliche Salze, dadurch gekennzeichnet, dass man

a) eine Verbindung der allgemeinen Formel II

$$Ar\text{-}Q\text{-}H \qquad (II),$$

in der

Ar und Q die oben aufgeführte Bedeutung haben, mit einer Verbindung der allgemeinen Formel III

$$X\text{-}A\text{-}Y \qquad (III),$$

in der

A die oben genannte Bedeutung hat und

X und Y reaktive Reste bedeuten,

wobei für den Fall, dass A für die Gruppe $-CH_2CH(OH)CH_2-$ steht, X-A-Y auch 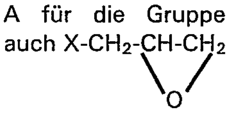 sein kann,

und einer Verbindung der allgemeinen Formel IV

$$R\text{-}S\text{-}H \qquad (IV),$$

in der

R die oben genannte Bedeutung hat,

umsetzt

oder

b) eine Verbindung der allgemeinen Formel V

$$Ar\text{-}Q\text{-}A\text{-}SH \qquad (V),$$

in der

Ar, Q und A die oben aufgeführte Bedeutung haben, mit einer Verbindung der allgemeinen Formel VI

$$R\text{-}X \qquad (VI),$$

in der

R und X die oben genannte Bedeutung haben, zur Reaktion bringt,

anschliessend gewünschtenfalls durch bekannte Verfahren den Schwefel oxidiert oder den Rest R in einen anderen Rest R umwandelt und das so erhaltene Reaktionsprodukt gewünschtenfalls in ein pharmakologisch verträgliches Salz überführt.

8. Arzneimittel, gekennzeichnet durch einen Gehalt an Verbindungen gemäss Ansprüche 1 bis 6 und übliche Träger- und Hilfsstoffe.

9. Verbindungen gemäss Ansprüche 1 bis 6 zur Verwendung in einem Verfahren zur Bekämpfung von allergischen Krankheiten.

**Claims**

1. Thioethers of the general formula I

$$\overset{(O)_n}{\uparrow}$$
$$Ar\text{-}Q\text{-}A\text{-}S\text{-}R \qquad (I),$$

in which

Ar signifies a phenyl radical which is substituted one to three times by alkyl, alkenyl, alkoxy, alkylthio, alkanoyl, hydroxyl, hydroxyalkyl, carboxyl, alkoxycarbonyl, carabamoyl, nitro, amino or halogen,

Q an oxygen atom, a sulphur atom or an NH group, A a straight-chained or branched $C_2$-$C_8$ alkylene radical which can optionally be substituted by a hydroxyl group or interrupted by an ethenylene or ethynylene group,

n is the number 0, 1 or 2, and

R a straight-chained or branched alkyl radical which can optionally be substituted by one or more identical or different radicals consisting of alkoxycarbonyl, alkoxy, hydroxyl, carboxyl or optionally substituted carbamoyl or amino; as well as their pharmacologically acceptable salts.

2. Thioethers according to claim 1, in which Ar is a 4-acetyl-3-hydroxyphenyl, a 4-acetyl-2-allyl-3-hydroxyphenyl, a 4-acetyl-3-hydroxy-2-propylphenyl or a 2-carboxyphenyl radical.

3. Thioethers according to claim 1 or 2, in which Q represents an oxygen atom.

4. Thioethers according to claim 1, 2 or 3, in which A represents a $-CH_2CH_2CH_2-$ or $-CH_2-CH(OH)-CH_2-$ radical.

5. Thioethers according to claim 1, 2, 3 or 4, in which R represents a $-CH_2-COOH$, $-CH_2CH_2-COOH$, $-CH_2-CH(NH_2)-COOR$ or a $-CH_2CH_2OH$ radical.

6. Thioethers according to claim 1, 2, 3, 4 or 5, in which n represents the number 0.

7. Process for the preparation of thioethers of the general formula I

$$\overset{(O)_n}{\uparrow} \atop \text{Ar-Q-A-S-R} \qquad (I),$$

in which

Ar signifies a phenyl radical which is substituted one to three times by alkyl, alkenyl, alkoxy, alkylthio, alkanoyl, hydroxy, hydroxyalkyl, carboxyl, alkoxycarbonyl, carbamoyl, nitro, amino or halogen,

Q an oxygen atom, a sulphur atom or an NH group,

A a straight-chained or branched $C_2$-$C_8$ alkylene radical which can optionally be substituted by a hydroxyl group or interrupted by an ethenylene or ethynylene group,

n the number 0, 1 or 2 and

R a straight-chained or branched alkyl radical which can optionally be substituted by one or more identical or different radicals consisting of alkoxycarbonyl, alkoxy, hydroxyl, carboxyl or optionally substituted carbamoyl or amino, as well as of their pharmacologically acceptable salts,

characterised in that one

a) reacts a compound of the general formula II

$$\text{Ar-Q-H} \qquad (II),$$

in which

Ar and Q have the above-given meaning, with a compound of the general formula III

$$\text{X-A-Y} \qquad (III),$$

in which

A has the above-given meaning and X and Y signify reactive groups, whereby, when A stands for the group $-CH_2CH(OH)CH_2-$, X-A-Y can also be 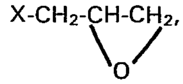 and a compound of the general formula IV

$$\text{R-S-H} \qquad (IV)$$

in which

R has the above-given meaning, or

b) brings a compound of the general formula V

$$\text{Ar-Q-A-SH} \qquad (V)$$

in which

Ar, Q und A have the above-given meaning, to reaction with a compound of the general formula VI

$$\text{R-X} \qquad (VI)$$

in which

R and X have the above-given meaning, subsequently, if desired, oxidises the sulphur by known processes or converts the radical R into another radical R and, if desired, converts the so-obtained reaction product into a pharmacologically acceptable salt.

8. Medicaments, characterised by a content of compounds according to claim 1 to 6 and usual carrier and adjuvant materials.

9. Compounds according to claims 1 to 6 for use in a process for the combating of allergic diseases.

**Revendications**

1. Thioéther de formule générale:

$$\overset{(O)_n}{\uparrow} \atop \text{Ar-Q-A-S-R} \qquad (I),$$

dans laquelle:

Ar est un reste phénylique qui est substitué de une à trois fois par un alkyle, alkényle, alcoxy, alkylthio, alcanoyle, hydroxyle, hydroxyalkyle, carboxyle, alcoxycarbonyle, carbamoyle, nitro, amino ou un halogène;

Q est un atome d'oxygène, un atome de soufre ou un groupe NH-;

A est un reste d'alkylène $C_{2-8}$ à chaîne droite ou ramifiée qui le cas échéant, peut être remplacé par un groupe hydroxyle ou interrompu par un groupe éthenylène ou éthinylène;

n est égal à 0, 1 ou 2; et,

R est un reste d'alkyle à chaîne droite ou ramifiée, qui, le cas échéant, peut être remplacé par un ou plusieurs restes identiques ou différents, consitués par de l'alcoxycarbonyle, de l'alcoxy, de l'hydroxyle, du carboxyle, ou, si on le désire, par du carbamoyle ou de l'amino substitués, ainsi que leurs sels utilisables en pharmacie.

2. Thioéther selon la revendication 1, dans lequel Ar est un reste de 4-acétyl-3-hydroxy-phényle, de 4-acétyl-2-allyl-3-hydroxy-phényle, de 4-acétyl-3--hydroxy-2-propoxy-phényle ou de 2-carboxy-phényle.

3. Thioéther selon l'une des revendications 1 ou 2, dans lequel Q représente un atome d'oxygène.

4. Thioéther selon l'une des revendications 1, 2 ou 3, dans lequel A représente un reste de $-CH_2-CH_2CH_2$ ou de $-CH_2CH(OH)-CH_2$.

5. Thioéther selon l'une des revendications 1, 2, 3 ou 4, dans lequel R représente un groupe $-CH_2-COOH$, $-CH_2CH_2-COOH-$, $-CH_2CH(NH_2)-COOH$, ou $-CH_2CH_2OH$.

6. Thioéther selon l'une des revendications 1, 2, 3, 4 ou 5, dans lequel n est égal à zéro.

7. Procédé de fabrication de thioéthers de formule générale:

$$\begin{matrix}(O)_n\\ \uparrow\\ \text{Ar-Q-A-S-R}\end{matrix} \qquad (I),$$

dans laquelle:

Ar est un reste phénylique qui est substitué de une à trois fois par un alkyle, alkényle, alcoxy, alkylthio, alcanoyle, hydroxyle, hydroxyalkyle, carboxyle, alcoxycarbonyle, carbamoyle, nitro, amino ou un halogène;

Q est un atome d'oxygène, un atome de soufre ou un groupe NH-;

A est un reste d'alkylène $C_{2-8}$ à chaîne droite ou ramifiée, qui, le cas échéant, peut être remplacé par un groupe hydroxyle ou interrompu par un groupe éthenylène ou éthinylène;

n est égal à 0, 1 ou 2; et,

R est un reste d'alkyle à chaîne droite ou ramifiée, qui, le cas échéant, peut être remplacé par un ou plusieurs restes identiques ou différents, constitués par de l'alcoxycarbonyle, de l'alcoxy, de l'hydroxyle, du carboxyle, ou, si on le désire, par de carbamoyle ou de l'amino substitués, ainsi que leurs sels utilisables en pharmacie

caractérisé en ce que l'on fait réagir:

a) un composé de formule générale II:

$$\text{Ar-Q-H} \qquad (II)$$

dans laquelle:

Ar et Q représentent les constituants déjà indiqués, avec un composé de formule générale III:

$$\text{X-A-Y} \qquad (III)$$

dans laquelle:

A représente le constituant déjà indiqué;

X et Y sont des restes réactifs,

étant entendu que, si A représente le groupe $-CH_2CH(OH)CH_2-$ X-A-Y peut également être

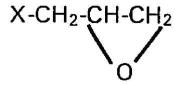

et, avec un composé de formule générale IV:

$$\text{R-S-H} \qquad (IV),$$

dans laquelle:

R représente les constituants déjà indiqués,

ou,

b) un composé ayant la formule générale V:

$$\text{Ar-Q-A-SH} \qquad (V),$$

dans alquelle:

Ar, Q et A représentent les constituants déjà indiqués, avec des composés de formule générale VI:

$$\text{R-X} \qquad (VI)$$

dans laquelle:

R et X représentent les constituants déjà indiqués, puis, si on le désire, on oxyde le soufre par des procédés connus, ou on transforme le reste R en un autre reste R, et on transforme le produit de réaction ainsi obtenu en un sel utilisable en pharmacie.

8. Médicament, caractérisé en ce qu'il contient des composés selon les revendications 1 à 6 et les supports ou additifs usuels.

9. Composés selon les revendications 1 à 6, destinés à être utilisés dans un traitement de lutte contre des maladies allergiques.